(19) Europäisches Patentamt
European Patent Office
Office européen des brevets

(11) Veröffentlichungsnummer : **0 414 059 B1**

(12) # EUROPÄISCHE PATENTSCHRIFT

(45) Veröffentlichungstag der Patentschrift : **27.10.93 Patentblatt 93/43**

(51) Int. Cl.$^5$ : **C07C 69/743,** C07C 67/08, A01N 37/08

(21) Anmeldenummer : **90115346.0**

(22) Anmeldetag : **10.08.90**

(54) **Dihalogenvinyl-benzylester, Verfahren und Vorprodukte zu ihrer Herstellung und ihre Verwendung zur Bekämpfung von Schädlingen.**

(30) Priorität : **19.08.89 DE 3927479**

(43) Veröffentlichungstag der Anmeldung : **27.02.91 Patentblatt 91/09**

(45) Bekanntmachung des Hinweises auf die Patenterteilung : **27.10.93 Patentblatt 93/43**

(84) Benannte Vertragsstaaten : **BE CH DE DK ES FR GB GR IT LI NL**

(56) Entgegenhaltungen :
**EP-A- 0 161 543**
**EP-A- 0 347 694**

(73) Patentinhaber : **BASF Aktiengesellschaft**
**Carl-Bosch-Strasse 38**
**D-67063 Ludwigshafen (DE)**

(72) Erfinder : **Wolf, Bernd, Dr.**
**Hallbergstrasse 4**
**D-6701 Fussgoenheim (DE)**
Erfinder : **Theobald, Hans, Dr.**
**Queichstrasse 6**
**D-6703 Limburgerhof (DE)**
Erfinder : **Hofmeister, Peter, Dr.**
**Bernard-Humblot-Strasse 12**
**D-6730 Neustadt (DE)**
Erfinder : **Kuenast, Christoph, Dr.**
**Salierstrasse 2**
**D-6701 Otterstadt (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Dihalogenvinyl-benzylester, Verfahren und Vorprodukte zu ihrer Herstellung, Schädlingsbekämpfungsmittel, die diese Ester als Wirkstoffe enthalten, sowie Verfahren zur Bekämpfung von Schädlingen mit diesen Wirkstoffen.

Es ist bekannt, daß 2,2-Dimethyl-3-(2',2'-dimethylvinyl)-cyclopropancarbonsäureester, die als Alkohol einen substituierten Vinylbenzylrest enthalten, insektizid wirksam sind (GB 2 065 475, BE 738 112).

Weiterhin werden in der europäischen Patentschrift EP-A 161 543 bestimmte Halogenvinylbenzylester zur Bekämpfung von Schädlingen offenbart. Die insektizide Wirksamkeit dieser Ester ist jedoch unter bestimmten Bedingungen (z.B. bei niedrigen Aufwandmengen) in den meisten Fällen unbefriedigend.

In der DE-A-38 20 895 werden Vinylbenzylester, die gegebenenfalls im Vinylteil alkylsubstituiert sind, als Pyrethroide beschrieben.

Es wurde nun gefunden, daß Dihalogenvinyl-benzylester der allgemeinen Formel I

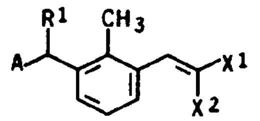

(I)

in der

- $R^1$ Wasserstoff, Cyano, $C_1$-$C_4$-Alkyl, $C_2$-$C_4$-Alkenyl oder $C_2$-$C_4$-Alkinyl,
- $X^1$ Chlor, Brom oder Fluor,
- $X^2$ Chlor, Brom oder Fluor und
- A den Carboxylat-Rest einer bei Pyrethroiden typischen Säurekomponente bedeuten,

starke insektizide und akarizide Wirkung aufweisen.

$C_1$-$C_4$-Alkyl steht z.B. für Methyl, Ethyl, Propyl, Isopropyl, n-Butyl oder iso-Butyl. $C_2$-$C_4$-Alkenyl steht z.B. für Vinyl, Allyl, Isopropenyl, 1-Butenyl oder 1-Methyl-1-propenyl. $C_2$-$C_4$-Alkinyl steht z.B. für Ethinyl oder 1-Propinyl.

Bevorzugt bedeuten in den Verbindungen I

- $R^1$ Wasserstoff,
- $X^1$ Chlor oder Brom,
- $X^2$ Chlor oder Brom und
- A die Carboxylat-Reste II, III oder IV.

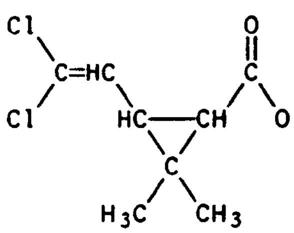

(II)

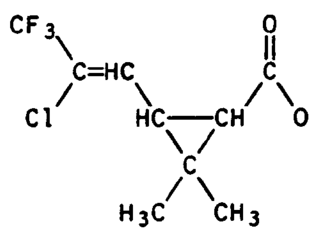

(III)

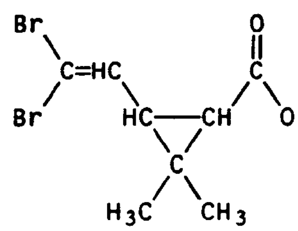

(IV)

Die Ester der allgemeinen Formel I können durch Umsetzung der Säuren A-H oder Derivaten dieser Sauren wie Säurehalogeniden, z.B. Säurechloride, Säureanhydriden, 1-Acyl-imidazolen o.ä. mit Benzylalkoholen der allgemeinen Formel V oder Derivaten von V wie z.B. Benzylchloriden oder Benzylbromiden nach folgendem Reaktionsschema gewonnen werden.

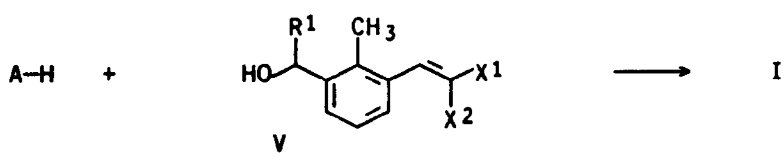

V

(Die Reste $R^1$, $X^1$, $X^2$ und A haben die in Anspruch 1 genannte Bedeutung).

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht im gewünschten Sinne verschoben werden, indem man dem Reaktionsgemisch das Wasser oder den Ester I entzieht, z.B. durch azeotrope Destillation oder durch Bindung des Wassers an Schwefel- oder Halogenwasserstoffsäure.

Ebensogut können die entsprechenden Säurechloride mit den Alkoholen der Formel V in Gegenwart eines Säureakzeptors umgesetzt werden (vgl. Houben-Weyl, Methoden der organ. Chemie, Band VIII, S. 541 ff, Georg-Thieme-Verlag, Stuttgart 1952).

Als Säurebindemittel eignen sich die üblichen basischen Mittel, insbesondere aliphatische, aromatische und heterocyclische Amine, z.B. Triethylamin, Dimethylamin, Piperidin, Dimethylanilin, Dimethylbenzylamin, Pyridin und 2-Picolin.

Die Umsetzung kann in einem Lösungs- oder Verdünnungsmittel ausgeführt werden. Hierzu sind die angeführten Säureakzeptoren selbst oder beispielsweise folgende Lösungs- oder Verdünnungsmittel oder Gemische derselben geeignet:

Alipatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von 0°C mit ausreichender Geschwindigkeit. Da sie meist unter Wärmeentwicklung verläuft, kann es von Vorteil sein, eine Kühlmöglichkeit vorzusehen.

In einigen Fällen ist es sinnvoll und vorteilhaft die Verbindungen der Formel V, insbesondere dann, wenn $R^1$ in der allgemeinen Formel V die Bedeutung einer Cyanogruppe hat, in situ zu verestern.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel V, durch Umsetzung von entsprechenden Salzen mit Derivaten der Alkohole der Formel V, wie z.B. den entsprechenden Benzylbromiden oder Benzylchloriden oder durch Umesterung (vgl. Houben-Weyl a.a.O. S. 508-628).

Die eingesetzten Pyrethroidsäuren und ihre Derivate sind beispielsweise in Wegler, Chemie der Pflanzenschutz- und Schädlingsbekämpfungsmittel, Band 7, Springer Verlag, Berlin, Heidelberg, New York 1981 beschrieben.

Typische Repräsentanten dieser Säuren der Formel A-H werden in der folgenden Aufzählung genannt, wobei diese Aufzählung keine Einschränkung bedeuten soll:

$A^1$-H: 3-(2',2'-Dimethylvinyl)-2,2-dimethylcyclopropan-1-carbonsäure
$A^2$-H: 3-(2',2'-Dichlorvinyl)-2,2-dimethylcyclopropan-1-carbonsäure
$A^3$-H: 3-(2'-Chlor-3',3',3'-trifluorprop-1'-enyl)-2,2-dimethylcyclopropan-1-carbonsäure
$A^4$-H: 3-(2',2'-Dibromvinyl)-2,2-dimethylcyclopropan-1-carbonsäure
$A^5$-H: 3-(2',2'-Difluorvinyl)-2,2-dimethylcyclopropan-1-carbonsäure
$A^6$-H: 3-(2'-Fluor-3',3',3'-trifluorprop-1'-enyl)-2,2-dimethylcyclopropan-1-carbonsäure
$A^7$-H: 3-(2',2'-Bistrifluormethyl-vinyl)-2,2-dimethylcyclopropan-1-carbonsäure
$A^8$-H: 2-(4'-Chlorphenyl)-3-methylbuttersäure
$A^9$-H: 2-(4'-Fluorphenyl)-3-methylbuttersäure
$A^{10}$-H: 2-(4'-Difluormethoxyphenyl)-3-methylbuttersäure
$A^{11}$-H: 3-(4'-tert.-Butylphenyl)-2,2-dimethylcyclopropan-1-carbonsäure
$A^{12}$-H: 2,2,3,3-Tetramethylcyclopropan-1-carbonsäure
$A^{13}$-H: 1-(4'-Chlorphenyl)-cyclopropan-1-carbonsäure
$A^{14}$-H: 1-(4'-Ethoxyphenyl)-2,2-dichlorcyclopropan-1-carbonsäure
$A^{15}$-H: 3-[2'-(4''-Chlorphenyl)-2'-chlorvinyl]-2,2-dimethylcyclopropan-1-carbonsäure
$A^{16}$-H: 3-(1',3'-Butadienyl)-2,2-dimethyl-cyclopropan-1-carbonsäure
$A^{17}$-H: 3-(2'-Methyl-2'-methoxycarbonyl-vinyl)-2,2-dimethylcyclopropan-1-carbonsäure
$A^{18}$-H: 2-(2'-Chlor-4'-trifluormethyl-phenylamino)-3-methylbuttersäure
$A^{19}$-H: 2-(2'-Fluor-4'-trifluormethyl-phenylamino)-3-methylbuttersäure
$A^{20}$-H: 3-Methyl-2-(4'-trifluormethyl-phenylamino)-buttersäure
$A^{21}$-H: 3-Methyl-2-(pyrrol-1'-yl)-buttersäure
$A^{22}$-H: 3-methyl-2-(3'-methylpyrrol-1'-yl)-buttersäure
$A^{23}$-H: 2-(3',4'-Dimethylpyrrol-1'-yl)-buttersäure

$A^{24}$-H: 2-(2',5'-Dimethylpyrrol-1'-yl)-3-methylbuttersäure
$A^{25}$-H: 2-(Isoindolin-2-yl)-3-methylbuttersäure
$A^{26}$-H: 1,1-Dimethyl-2,2[H]indenspirocyclopropan-3-carbonsäure
$A^{27}$-H: 3-Cyclopentylidenmethyl-2,2-dimethylcyclopropan-1-carbonsäure
$A^{28}$-H: 3-(1',2'-Dibrom-2',2'-dichlorethyl)-2,2-dimethylcyclopropan-1-carbonsäure

Es versteht sich, daß die Verbindungen der Formel I in jedem Falle in Form von einheitlichen Diastereomeren, mindestens einem Paar optischer Antipoden, in vielen Fällen auch in Form von Diastereomeren-Vielfachen vorkommen und als Wirkstoffe angewandt werden können, die je nach den Ausgangsstoffen und den Umsetzungsbedingungen in einheitlicher Form oder als Gemische auftreten. Die Gemische können in üblicher Weise in ihre sterisch einheitlichen Bestandteile aufgetrennt werden; ihre biologische Wirkung ist in Einzelfällen von ihrer sterischen Konfiguration abhängig.

Die als Ausgangsstoff benötigten Dihalogenvinyl-benzylalkohole der allgemeinen Formel V sind neu und können ausgehend von der käuflichen Nitroverbindung VI nach teilweise bekannten Methoden erhalten werden.

Folgendes Reaktionsschema, in dem die Abkürzungen THP für Tetrahydro-2-pyranyl und THF für Tetrahydrofuran stehen, soll den Syntheseweg verdeutlichen:

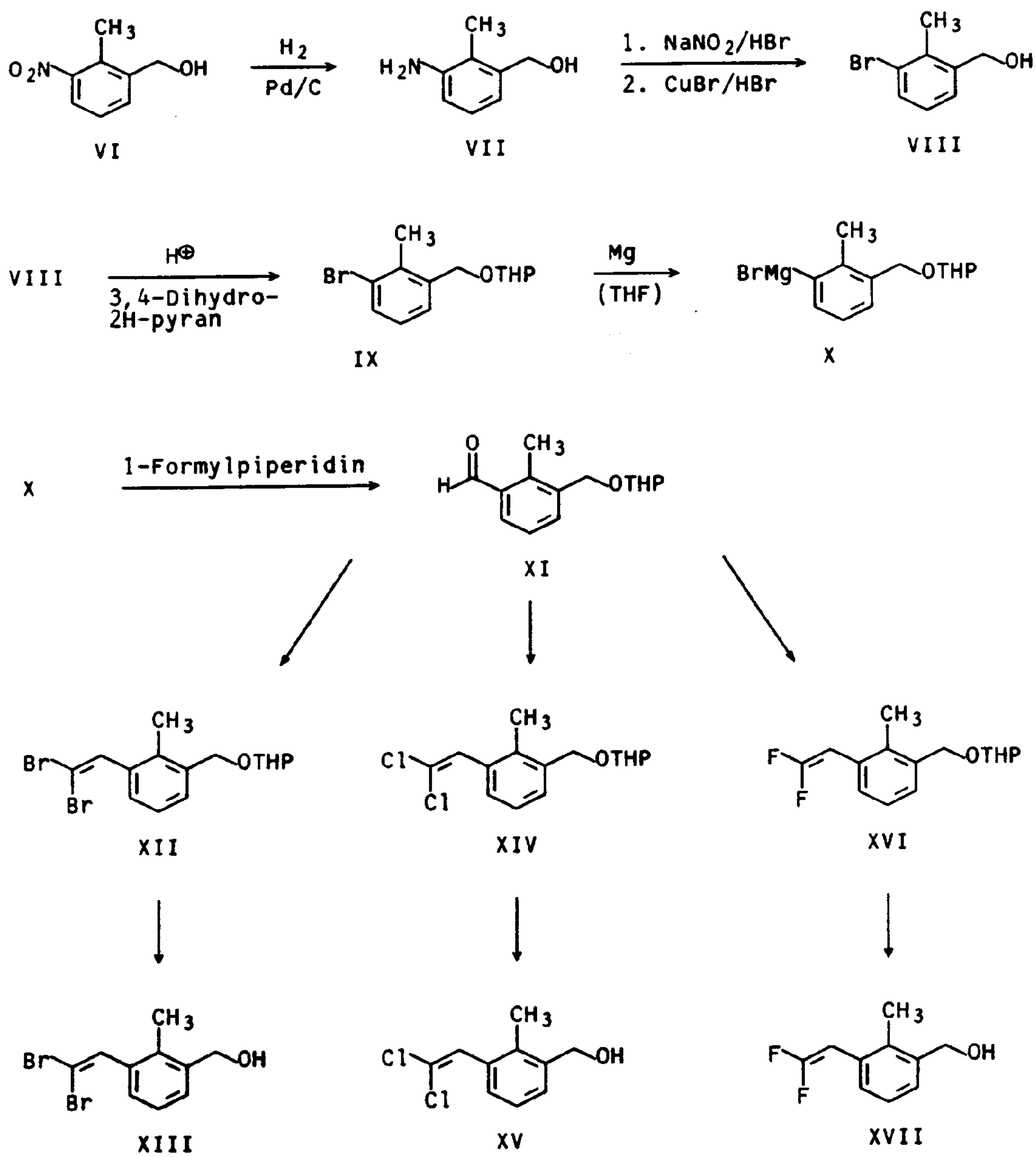


VI VII VIII

VIII IX X

X XI

XII XIV XVI

XIII XV XVII

Die Herstellung von 3-Amino-2-methylbenzylalkohol VII und 3-Brom-2-methylbenzylalkohol VIII kann nach den in EP-A 54 180 beschriebenen Verfahren erfolgen.

Der Alkohol VIII läßt sich nach der in Tietze/Eicher (Reaktionen und Synthesen, Thieme Verlag, 1981, Seite 184) beschriebenen Methode in den Tetrahydropyranylether IX umwandeln.

Zur Herstellung des Aldehyds XI kommt die Umsetzung der entsprechenden metallorganischen Verbindungen (Grignardverbindung oder Lithiumorganylverbindung) mit bestimmten Formamiden wie z.B. Dimethylformamid, 1-Formylpiperidin oder 2-(Formyl-methylamino)-pyridin in Frage (vgl. Houben-Weyl, Methoden der organischen Chemie, Band E 3, S. 130).

Die Dibromvinylverbindung XII kann nach der in Liebigs Ann. Chem. 1980, 2061-2071 beschriebenen Methode hergestellt werden. Zur Herstellung der entsprechenden Dichlorvinylverbindung XIV eignet sich die Umsetzung des Aldehyds mit Triphenylphosphin/Chloroform in Gegenwart einer Base (Org. Synth. Vol. 5, Seite 361) oder mit Tetrachlorkohlenstoff/Triphenylphosphin [J. Am. Chem. Soc. 84, 1312 (1962)]. Besonders vorteilhaft lassen sich Dihalogenvinylverbindungen durch Reaktion von Aldehyden mit Trichlormethylphosphonsäurediestern/n-Butyllithium herstellen (Synthesis 1975, S. 458).

Das Difluorvinylderivat XVI kann nach der in Synthesis 1965, S. 1027 beschriebenen Methode durch Umsetzung des Aldehyds mit Triphenylphosphin und Natrium-chlordifluoracetat gewonnen werden.

Zur Abspaltung der Tetrahydropyranyl-Schutzgruppe eignen sich die in Tietze/Eicher (Reaktionen und Synthesen, Georg Thieme Verlag 1981, S. 363) beschriebenen Verfahren.

Die Benzylalkohole der allgemeinen Formel V, in der $R^1$ Cyano, $C_2$-$C_4$-Alkinyl, $C_2$-$C_4$-Alkenyl oder $C_1$-$C_4$-Alkyl bedeutet, erhält man vorteilhaft dadurch, daß man zunächst die unsubstituierten Benzylalkohole mit $R^1$=H (Va) zu den entsprechenden Benzaldehyden XVIII oxidiert.

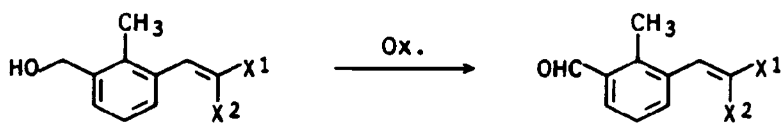


Va XVIII

Als Oxidationsmittel kommen alle gängigen Oxidationsmittel, die primäre Alkohole in Aldehyde überführen, in Frage (Houben-Weyl, Methoden der organischen Chemie, Band E3, S. 265 ff). Besonders geeignet sind Verbindungen mit Übergangsmetallen in höherer Oxidationsstufe, z.B. Pyridiniumchlorochromat.

Die Benzaldehyde XVIII können in einem anschließenden Reaktionsschritt in an sich bekannter Weise zu den substituierten Benzylalkoholen mit $R^1 \neq H$ umgesetzt werden. Dabei wird

a) im Fall von $R^1$ = CN der Benzaldehyd mit Blausäure oder einem Metallcyanid ggf. in Gegenwart einer Säure umgesetzt;

b) im Fall von $R^1$ = $C_2$-$C_4$-Alkinyl, $C_2$-$C_4$-Alkenyl oder $C_1$-$C_4$-Alkyl, der Benzaldehyd mit Metallorganylen $MeR^1$ oder $R^1MeHal$, wobei Me für Alkali-, Erdalkali- oder Übergangsmetalle und Hal für Halogen steht, zur Reaktion gebracht.

Zur Herstellung der Cyanhydrine werden die Benzaldehyde mit Blausäure, in situ aus Metallcyaniden erzeugter Blausäure oder Metallcyaniden in Gegenwart von Alkalihydrogensulfitlösung umgesetzt, wobei gegebenenfalls basische Katalysatoren wie Kaliumcarbonat oder Phasentransferkatalysatoren wie z.B. Benzyltriethylammoniumchlorid zugegeben werden.

Als Metallcyanide werden bevorzugt Alkalimetallcyanide wie z.B. Natrium- oder Kaliumcyanid verwendet.

Die Umsetzung erfolgt in an sich bekannter Weise, z.B. wie in Houben-Weyl, Methoden der org. Chemie, Band VIII, S. 274-278, Ausgabe 1952 und Band E5, S. 1413 ff, 1985, beschrieben.

Als Metallorganyle eignen sich die entsprechenden metallorganischen Verbindungen, insbesondere Lithiumorganylverbindungen $LiR^1$ wie Methyllithium, Ethyllithium, Butyllithium oder die entsprechenden Grignardverbindungen $R^1MgHal$, worin Hal für Chlor, Brom oder Jod steht, z.B. Methylmagnesiumbromid, Ethylmagnesiumchlorid, Propylmagnesiumiodid oder Vinylmagnesiumiodid.

Die Umsetzung mit Metallorganylen kann in an sich bekannter Weise, z.B. wie in Houben-Weyl, Methoden der org. Chemie, Band 13/2a, S. 285ff, 1973 beschrieben in einem inerten organischen Lösungsmittel wie Ether oder Tetrahydrofuran unter Schutzgas durchgeführt werden, so daß sich nähere Ausführungen hierzu erübrigen.

Die Herstellung der erfindungsgemäßen Verbindungen erfolgt gemäß den Beschreibungen der folgenden Beispiele bzw. durch entsprechende Abwandlung dieser Beispiele.

Herstellbeispiel 1

cis,trans-3-(2'-Chlor-3',3',3'-trifluorprop-1'-enyl)-2,2-dimethylcyclopropan-1-carbonsäure-[3-(2',2'-dichlorvi nyl)-2-methylbenzyl]ester

1,9 g 3-(2',2'-Dichlorvinyl)-2-methylbenzylalkohol und 0,95 g 2-Picolin in 20 ml Toluol werden bei 20°C tropfenweise mit 2,61 g 3-(2'-Chlor-3',3',3'-trifluorprop-1'-enyl)-2,2-dimethylcyclopropan-1-carbonsäurechlorid (cis/trans=1:1) versetzt. Nach dem Abklingen der exothermen Reaktion läßt man 5 Stunden nachreagieren, gießt die Reaktionsmischung über eine mit Kieselgel gefüllte Nutsche und spült mit Toluol nach. Nach Abdestillieren des Lösungsmittels erhält man 3,9 g eines hellgelben Öls.

200-MHz-NMR-Spektrum in $CDCl_3$:

$\delta$ [ppm] = 1,21-1,37 (6H); 1,83; 2,03; 2,16 und 2,44 (2H); 2,28 (3H); 5,18 (2H); 6,15 und 6,95 (1H); 6,97 (1H); 7,21-7,46 (3H).

Herstellbeispiel 2

cis-3-(2',2'-Dichlorvinyl)-2,2-dimethylcyclopropan-1-carbonsäure-[3-(2',2'-dibromvinyl)-2-methylbenzyl]ester und trans-3-(2',2'-Dichlorvinyl)-2,2-dimethylcyclopropan-1-carbonsäure-[3-(2', 2'-dibromvinyl)-2-methylbenzyl]ester

0,66 g Kaliumhydroxid werden in 30 ml Ethanol gelöst und die Lösung wird mit 2,09 g 3-(2',2'-Dichlorvinyl)-2,2-dimethylcyclopropan-1-carbonsäure (cis/trans=1:1) versetzt. Das Lösungsmittel wird abdestilliert und das erhaltene Kaliumsalz der Säure in 50 ml Dimethylformamid gelöst. Man tropft 3,69 g 3-(2',2'-Dibromvinyl)-2-methylbenzylbromid hinzu und rührt 3 Stunden bei 120°C. Die Reaktionsmischung gießt man in 250 ml Wasser und extrahiert mit Ether. Die vereinigten organischen Phasen werden mit Wasser gewaschen, getrocknet und eingeengt. Das erhaltene Rohprodukt wird säulenchromatographisch über Kieselgel mit Toluol/Cyclohexan (3/7) gereinigt und gleichzeitig in den cis- und trans-Benzylester aufgetrennt (Beispiel 2a = cis-Verbindung, Beispiel 2b = trans-Verbindung)

300-MHz-NMR-Spektrum in $CDCl_3$:

cis-Verbindung (2a):

δ [ppm] = 1,25 (6H); 1,89 (1H); 2,04 (1H); 2,25 (3H); 5,13 (2H); 6,27 (1H); 7,23 (1H); 7,33 (2H): 7,5 (1H);

trans-Verbindung (2b):

δ [ppm] = 1,16 (3H); 1,27 (3H); 1,66 (1H); 2,25 (3H + 1H); 5,16 (2H); 5,62 (1H); 7,23 (1H); 7,34 (2H); 7,5 (1H).

Herstellbeispiel 3

3-(2',2'-Dichlorvinyl)-2-methylbenzylalkohol

2,1 g (3-(2',2'-Dichlorvinyl)-2-methylbenzyl]-(tetrahydro-2-pyranyl)ether in 20 ml Methanol werden mit 1,12 ml konzentrierter Salzsäure 15 Stunden bei Raumtemperatur gerührt. Unter Eiskühlung wird mit Natriummethylat-Lösung neutralisiert und anschließend eingeengt. Den Rückstand versetzt man mit Wasser und extrahiert mehrmals mit Ether. Die vereinigten Etherextrakte werden mit Wasser gewaschen und getrocknet. Nach Abdestillieren des Lösungsmittels erhält man 1,5 g 3-(2',2'-Dichlorvinyl)-2-methylbenzylalkohol.

300-MHz-NMR-Spektrum in $CDCl_3$:

δ [ppm] = 2,04 (1H); 2,23 (3H); 4,65 (2H); 6,93 (1H); 7,22 (1H); 7,34 (2H);

Herstellbeispiel 4

3-(2',2'-Dibromvinyl)-2-methylbenzylalkohol

2,93 g [3-(2',2'-Dibromvinyl)-2-methylbenzyl]-(tetrahydro-2-pyranyl)ether in 30 ml Methanol werden mit 1,2 ml konzentrierter Salzsäure 2 Tage lang bei Raumtemperatur gerührt. Unter Eiskühlung wird mit Natriummethylat-Lösung neutralisiert und anschließend eingeengt. Den Rückstand versetzt man mit Wasser und extrahiert mehrmals mit Ether. Die vereinigten Etherextrakte werden mit Wasser gewaschen, getrocknet und eingeengt. Man erhält 2,4 g 3-(2',2'-Dibromvinyl)-2-methylbenzylalkohol (Smp.: 63-68°C).

300-MHz-NMR-Spektrum in $CDCl_3$:

δ [ppm] = 2,13 (1H); 2,22 (3H); 4,65 (2H); 7,15-7,35 (3H); 7,47 (1H).

Herstellung von Ausgangsprodukten:

Herstellbeispiel 5

3-Amino-2-methylbenzylalkohol

Eine Lösung von 203 g 2-Methyl-3-nitrobenzylalkohol in 1 000 ml Ethanol wird in Gegenwart von 5 g Pd/Kohle (10 %ig) bei 30-35°C hydriert. Der Katalysator wird abfiltriert, die Lösung eingeengt und der erhaltene Feststoff getrocknet. Man erhält 145 g 3-Amino-2-methylbenzylalkohol (Smp. 104-108°C).

Herstellbeispiel 6

3-Brom-2-methylbenzylalkohol

Zu einem auf 0°C abgekühlten Gemisch von 888 ml Wasser, 162 ml Bromwasserstoffsäure (47 %ig) und 81,2 g 3-Amino-2-methylbenzylalkohol wird eine Lösung von 40,7 g Natriumnitrit in 300 ml Wasser getropft. Man rührt 30 Minuten bei 0°C und gibt dann bei dieser Temperatur portionsweise eine Suspension von 168,7 g Kupfer(I)-bromid in 750 ml Wasser hinzu. Das Reaktionsgemisch wird nacheinander 1 Stunde bei 10°C, 1 Stunde bei Raumtemperatur und 2 Stunden bei 100°C gerührt. Nach dem Abkühlen wird das Reaktionsgemisch

mehrmals mit Ether extrahiert. Die vereinigten organischen Phasen werden mit Wasser gewaschen, getrocknet und eingeengt. Nach säulenchromatographischer Reinigung über Kieselgel mit Toluol als Fließmittel erhält man 64,2 g 3-Brom-2-methylbenzylalkohol (Smp. 97-100°C).

Herstellbeispiel 7

(3-Brom-2-methylbenzyl)-(tetrahydro-2-pyranyl)ether

Zu einer Lösung von 208,9 g 3-Brom-2-methylbenzylalkohol und 87,4 g 3,4-Dihydro-2H-pyran in 1 600 ml Ether werden bei 0°C 2,1 ml konzentrierte Salzsäure gegeben. Man rührt 4 Tage lang bei Raumtemperatur und tropft dann 50 ml 10 %ige Kalilauge und 500 ml Wasser hinzu. Die organische Phase wird abgetrennt, die wäßrige Phase mit Ether extrahiert und die vereinigten organischen Phasen werden mit Wasser gewaschen, getrocknet und eingeengt. Nach säulenchromatographischer Reinigung über Kieselgel mit Toluol als Fließmittel erhält man 224,6 g der gewünschten Verbindung.

300-MHz-NMR-Spektrum in $CDCl_3$:

$\delta$ [ppm] = 1,4-1,91 (6H); 2,42 (3H); 3,55 (1H); 3,89 (1H); 4,47 (1H); 4,68 (1H); 4,8 (1H); 7,02 (1H); 7,32 (1H); 7,47 (1H).

Herstellbeispiel 8

(3-Formyl-2-methylbenzyl)-(tetrahydro-2-pyranyl)ether

Unter Stickstoffatmosphäre werden 1,2 g Mg (Magnesiumspäne) in 15 ml absolutem Tetrahydrofuran vorgelegt. Bei 65°C fügt man einige Tropfen Dibromethan hinzu. Während man die Temperatur weiterhin bei 65°C hält wird eine Lösung von 14,25 g (3-Brom-2-methylbenzyl)-(tetrahydro-2-pyranyl)-ether in 50 ml Tetrahydrofuran zugetropft. Anschließend rührt man 2 Stunden unter Rückfluß. Zu der auf 0°C abgekühlten Reaktionsmischung wird eine Lösung von 5,65 g N-Formylpiperidin in 10 ml absolutem Tetrahydrofuran getropft. Man rührt 20 Stunden bei Raumtemperatur, stellt mit ca. 50 ml 5 %iger Salzsäure schwach sauer und extrahiert mehrmals mit Ether. Die vereinigten Etherextrakte werden mit Wasser gewaschen, getrocknet und eingeengt. Das Rohprodukt (11 g) kann säulenchromatographisch über Kieselgel mit Toluol/Aceton (97,5:2,5) gereinigt werden.

250-MHz-NMR-Spektrum in $CDCl_3$:

$\delta$ [ppm] = 1,48-1,93 (6H); 2,64 (3H); 3,57 (1H); 3,9 (1H); 4,54 (1H); 4,72 (1H); 4,86 (1H); 7,36 (1H); 7,65 (1H); 7,76 (1H); 10,33 (1H).

Herstellbeispiel 9

[3-(2',2'-Dibromvinyl)-2-methylbenzyl]-(tetrahydro-2-pyranyl) ether

Zu einer Lösung von 64,85 g Triphenylphosphin in 60 ml Methylenchlorid wird bei 0°C eine Lösung von 41,44 g Tetrabrommethan in 40 ml Methylenchlorid getropft. Man rührt 30 Minuten bei 0°C und tropft dann 23,1 g (3-Formyl-2-methylbenzyl)-(tetrahydro-2-pyranyl)ether in 25 ml Methylenchlorid hinzu. Nach 2stündigem Rühren bei Raumtemperatur wird der Feststoff abfiltriert und das Filtrat eingeengt. Zum Filtrat werden 200 ml Cyclohexan und 200 ml Wasser hinzugegeben. Man rührt 1 Stunde unter Rückfluß, trennt die organische Phase ab, trocknet und engt ein. Nach säulenchromatographischer Reinigung über Kieselgel mit Cyclohexan und Toluol als Fließmittel erhält man neben 4 g 3-(2',2'-Dibromvinyl)-2-methylbenzylbromid 18,4 g der gewünschten Verbindung.

300-MHz-NMR-Spektrum in $CDCl_3$:

$\delta$ [ppm] = 1, 48-1, 92 (6H); 2,23 (3H); 3,55 (1H); 3,9 (1H); 4,48 (1H); 4,72 (1H); 4,81 (1H); 7,19 (1H); 7,28 (1H); 7,38 (1H); 7,5 (1H).

Herstellbeispiel 10

[3-(2',2'-Dichlorvinyl)-2-methylbenzyl]-(tetrahydro-2-pyranyl)ether

Methode A:

Zu einer Suspension von 3,37 g Kalium-tert.-butylat in 50 ml Heptan werden unter Stickstoffatmosphäre bei 0-5°C zügig 7,86 g Triphenylphosphin gegeben. Anschließend werden ebenfalls bei 0-5°C 3,59 g Chloroform in 30 ml Heptan innerhalb von 1 Stunde zugetropft. Das entstandene tert.-Butanol wird bei 0°C unter vermindertem Druck abdestilliert. Bei 5-10°C tropft man innerhalb von 30 Minuten eine Lösung von 7,02 g (3-Formyl-2-methylbenzyl)-(tetrahydro-2-pyranyl)ether in 10 ml Heptan ein, rührt 2 Stunden bei 5°C und 15 Stunden bei Raumtemperatur. Der ausgefallene Feststoff wird abfiltriert und die Lösung eingeengt. Nach säulenchromatographischer Reinigung über Kieselgel mit Toluol als Fließmittel erhält man 2,3 g des gewünschten Ethers.

Methode B:

Zu einer Lösung von 12,8 g Trichlormethyl-phosphonsäure-diethylester in 45 ml Ether und 35 ml Tetrahydrofuran werden unter Stickstoffatmosphäre bei -100°C 33,1 ml (0,053 mol) n-Butyllithium (15 %ige Lösung in Hexan) und anschließend 11,7 g (3-formyl-2-methylbenzyl)-(tetrahydro-2-pyranyl)ether in 10 ml Ether/Tetrahydrofuran (1:1) getropft. Unter Rühren läßt man auf Raumtemperatur aufwärmen und erhitzt 1 Stunde unter Rückfluß. Die Reaktionsmischung wird auf -50°C abgekühlt, mit 50 ml 2N Schwefelsäure versetzt, in 300 ml Wasser gegossen und mehrmals mit Ether extrahiert. Die vereinigten Etherphasen werden mit Wasser gewaschen, getrocknet und eingeengt. Nach säulenchromatographischer Reinigung über Kieselgel mit Toluol als Fließmittel erhält man 4,7 g der gewünschten Verbindung.
300-MHz-NMR-Spektrum in $CDCl_3$:
δ [ppm] = 1,47-1,93 (6H); 2,25 (3H); 3,57 (1H); 3,92 (1H); 4,48 (1H); 4,72 (1H); 4,81 (1H); 6,95 (1H); 7,2 (1H); 7,35 (2H).

Nach den beschriebenen Verfahren wurden die in der folgenden Tabelle genannten erfindungsgemäßen Ester hergestellt, die durch die Angabe physikalischer Eigenschaften näher charakterisiert sind; die übrigen Verbindungen der Tabelle können unter Verwendung entsprechender Ausgangsstoffe leicht erhalten werden:

Tabelle

Dihalogenvinyl-benzylester der Formel I

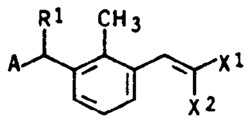

(I)

| Nr. | $A^{n}$*) | $X^1$ | $X^2$ | $R^1$ | NMR-Daten (MHz, LM) δ [ppm] |
|---|---|---|---|---|---|
| 11 | $A^4$ | Cl | Cl | H | |
| 12 | $A^5$ | Cl | Cl | H | |
| 13 | $A^8$ | Cl | Cl | H | |
| 14 | $A^{18}$ | Cl | Cl | H | |
| 15 | $A^2$ | Cl | Cl | Ethinyl | |
| 16 | $A^2$ | Cl | Cl | H | (200, $CDCl_3$);1,18-1,33(6H);1,67;1,9;2,05 und 1 teilweise verdecktes Signal(2H);2,27(3H); 5,17(2H);5,64 und 6,3(1H);6,97(1H); 7,21-7,46(3H) |
| 17 | $A^2$ | Cl | Cl | CN | |
| 18 | $A^2$ | Cl | Cl | Vinyl | |
| 19 | $A^1$ | Cl | Cl | H | |
| 20 | $A^{28}$ | Cl | Cl | H | |
| 21 | $A^2$ | Cl | Cl | $CH_3$ | |
| 22 | $A^3$ cis | Br | Br | H | (300, $CDCl_3$);1,28(6H);2,03(1H);2,18(1H); 2,24(3H);5,14(2H);6,93(1H);7,22(1H); 7,34(2H);7,5(1H) |
| 23 | $A^3$ | Br | Br | CN | |
| 24 | $A^3$ | Br | Cl | H | |
| 25 | $A^2$ | Cl | F | H | |
| 26 | $A^2$ | Br | Br | Ethinyl | |
| 27 | $A^5$ | Br | Br | H | |
| 28 | $A^3$ trans | Br | Br | H | (300, $CDCl_3$);1,23(3H);1,33(3H);1,82(1H); 2,26(3H);2,43(1H);5,19(2H);6,14(1H); 7,23(1H);7,35(2H);7,5(1H); |
| 29 | $A^1$ | Br | Br | $CH_3$ | |
| 30 | $A^5$ | Br | Br | H | |
| 31 | $A^{12}$ | Br | Br | H | |
| 32 | $A^{15}$ | Br | Br | H | |
| 33 | $A^6$ | Br | Br | H | |
| 34 | $A^4$ | Br | Br | H | (300, $CDCl_3$);1,18-1,3(6H);1,68;1,88;1,97 und 1 teilweise verdecktes Signal(2H);2,25(3H); 5,15(2H);6,16 und 6,77(1H);7,23(1H); 7,34(2H);7,5(1H) |

Tabelle (Fortsetzung)

| Nr. | $A^{n*)}$ | $X^1$ | $X^2$ | $R^1$ | NMR-Daten (MHz, LM) δ [ppm] |
|---|---|---|---|---|---|
| 35 | $A^2$ | Br | Br | Vinyl | |
| 36 | $A^2$ | F | F | H | |
| 37 | $A^3$ | F | F | H | |
| 38 | $A^4$ | F | F | H | |
| 39 | $A^8$ | F | F | H | |
| 40 | $A^8$ | Br | Br | H | (300, $CDCl_3$);0,67(3H);0,99(3H);2,1(3H); 2,33(1H);3,17(1H);5,09(2H);7,13-7,34(7H); 7,47(1H) |
| 41 | $A^{10}$ | F | F | H | |
| 42 | $A^{28}$ | Br | Br | H | |
| 43 | $A^2$ | F | F | CN | |
| 44 | $A^3$ | F | F | Ethinyl | |
| 45 | $A^{19}$ | Br | Br | H | |
| 46 | $A^{18}$ | Br | Br | H | (300, $CDCl_3$);1,02(6H);2,2(3H + 1H);3,96(1H); 5,07(1H); 5,19(2H);6,56(1H);7,16-7,54(6H) |
| 47 | $A^{18}$ | F | F | H | |
| 48 | $A^{15}$ | Cl | Cl | H | |
| 49 | $A^{12}$ | Cl | Cl | H | |
| 50 | $A^3$ | Cl | F | H | |
| 51 | $A^{20}$ | Cl | Cl | H | |
| 52 | $A^{20}$ | Br | Br | H | (300, $CDCl_3$);1,01(6H);2,18(3H+1H);3,94(1H); 4,46(1H);5,18(2H);6,61(2H);7,14-7,42(5H); 7,47(1H) |
| 53 | $A^{20}$ | F | F | H | |
| 54 | $A^5$ | F | F | H | |
| 55 | $A^9$ | Br | Br | H | |
| 56 | $A^{10}$ | Cl | Cl | H | |

*) $A^n$ (n=1-28) steht für den jeweiligen Carboxylat-Rest einer im vorstehenden Text genannten Pyrethroidsäure ($A^n$-H)

Die substituierten Dihalogenvinyl-benzylester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana,- Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera

bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis,

Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 16 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 22 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 28 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 40 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 52 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,1 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Anwendungsbeispiele

In den nachfolgenden Beispielen wurden die erfindungsgemäßen Verbindungen mit den aus EP-A 161 543 bekannten Verbindungen A bis D verglichen:

A:

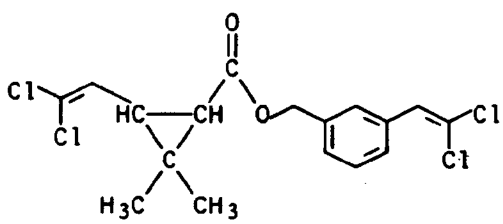

B:

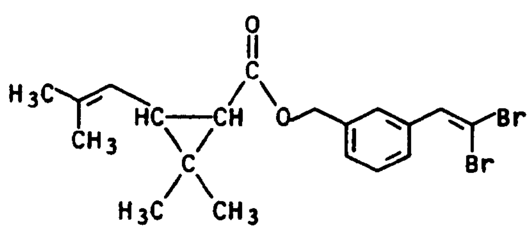

C:

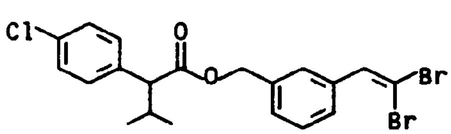

D:

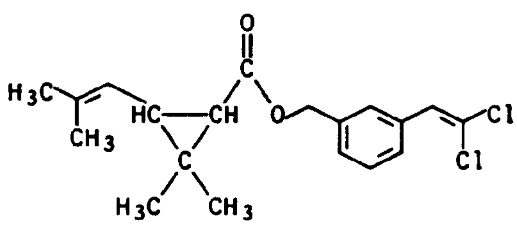

Die Reinheit der Wirkstoffe wie der Vergleichsmittel lag bei >95 %. Die Wirkstoffe wurden entweder in Form einer 0,1 %igen acetonischen Lösung oder eines Emulsionskonzentrates, das 10 Gew.% Wirkstoff enthält, verwendet.

Das Emulsionskonzentrat wird durch Emulgieren des Wirkstoffs in einer Mischung, enthaltend 70 Gew.% Cyclohexanon, 20 Gew.% Nekanil® LN (≙ Lutensol AP6, Netzmittel mit Emulgier- und Dispergierwirkung auf Basis ethoxylierter Alkylphenole), 10 Gew.% Emulphor® EL (≙ Emulan EL®, Emulgator auf Basis ethoxylierter Fettalkohole) hergestellt. Die in den Beispielen angegebenen Konzentrationen wurden durch Verdünnen des formulierten Wirkstoffes mit Wasser erhalten.

Kontaktwirkung auf Blatta orientalis (Schabe)

Der Boden eines 1 l-Glases wird mit der acetonischen Lösung des Wirkstoffs behandelt. Nach Verdunsten des Lösungsmittels setzt man in jedes Glas 3 adulte Schaben. Die Mortalitätsrate wird nach 48 Stunden bestimmt.

In diesem Versuch zeigten die Vergleichsverbindungen A bis D keine Wirkung bei einer Konzentration von 1 mg und darunter, während die Wirkstoffe 1, 2a, 2b, 16, 22, 28, 34, 40 eine Mortalitätsrate von 60 bis 100 % im Konzentrationsbereich von 0,04 bis 0,2 mg aufwiesen.

Kontaktwirkung auf Sitophilus granaria (Kornkäfer)

Man gibt 1 ml des in Aceton gelösten Wirkstoffs in Petrischalen (Φ 10 cm), deren Rand mit einer wäßrigen Suspension von Polytetrafluorethylen (Fluon®) behandelt ist, läßt das Lösungsmittel verdunsten und setzt in jede Schale 50 Kornkäfer.

Nach 4 Stunden werden die Käfer in Pappschälchen (Φ 40 mm, Höhe 10 mm) umgesetzt und in die Petrischalen zurückgestellt (Käfer, die das Pappschälchen nicht mehr verlassen können, gelten als tot bzw. schwer geschädigt). Die Wirkung bestimmt man nach 24 Stunden in Mortalität.

In diesem Versuch zeigten die Vergleichsverbindungen A bis D keine Wirkung bei einer Konzentration von 1 mg und darunter, während die Wirkstoffe 1, 2a, 2b, 16, 22, 28, 34, eine Mortalitätsrate von 80 bis 100 % im Konzentrationsbereich von 0,04 bis 1 mg aufwiesen.

Kontaktwirkung auf Aedes aegypti (Gelbfiebermücke)

200 ml Leitungswasser in 250 ml Plastikbechern werden mit der Wirkstoffaufbereitung versetzt und 500 bis 100 Moskitolarven im 3.-4. Larvenstadium hinzugegeben. Die Versuchstemperatur beträgt 25°C. Nach 24 Stunden wird die Wirkung in % Mortalität ermittelt.

In diesem Versuch zeigten die Wirkstoffe 1, 2a, 2b, 16, 22, 28 und 34 eine weitaus bessere Wirkung als die Vergleichssubstanzen B bis D.

Dauerkontakt auf Musca domestica (Stubenfliege)

In beide Teile einer Petrischale von 10 cm Durchmesser gibt man insgesamt 2 ml der acetonischen Wirkstofflösung, läßt das Lösungsmittel (ca. 30 Minuten) verdunsten und setzt je 10 Fliegen in die Schalen. Die Wirkung in % Mortalität wird nach 4 Stunden ermittelt.

In diesem Versuch zeigten die Wirkstoffe 1, 2a, 2b, 16, 22, 28, 34 und 40 eine deutlich bessere Wirkung als die Vergleichssubstanzen A bis D.

Kontaktwirkung auf Plutella maculipennis (Kohlschaben-Raupe)

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird dann mit 5 Raupen des 4. Stadiums belegt. Nach 48 Stunden beurteilt man die Mortalität in %.

In diesem Versuch zeigten die Wirkstoffe 1, 2a, 2b, 16, 22, 28 und 34 eine weitaus bessere Wirkung als die Vergleichssubstanzen A bis D.

## Patentansprüche

1. Dihalogenvinyl-benzylester der allgemeinen Formel I

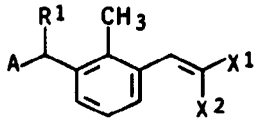

(I)

in der

$R^1$ Wasserstoff, Cyano, $C_1$-$C_4$-Alkyl, $C_2$-$C_4$-Alkenyl oder $C_2$-$C_4$-Alkinyl,

$X^1$ Chlor, Brom oder Fluor,

$X^2$ Chlor, Brom oder Fluor und

A den Carboxylat-Rest einer bei Pyrethroiden typischen Säurekomponente bedeuten.

2. Verbindungen gemäß Anspruch 1, dadurch gekennzeichnet, daß $R^1$ für Wasserstoff steht.

3. Verbindungen gemäß den Ansprüchen 1 und 2, dadurch gekennzeichnet, daß A für die Carboxylat-Reste II, III oder IV steht.

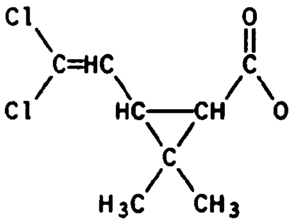

(II)

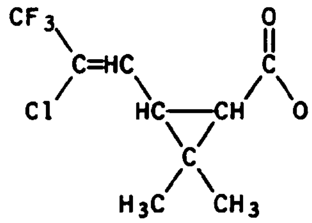

(III)

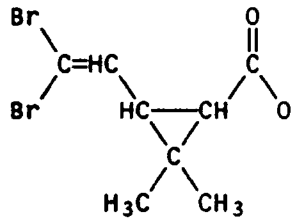

(IV)

4. Verfahren zur Herstellung von Verbindungen nach den Ansprüchen 1 bis 3, dadurch gekennzeichnet, daß man bei Pyrethroiden typische Säuren der allgemeinen Formel A-H oder Säurederivate hiervon mit Verbindungen der allgemeinen Formel V oder Derivaten dieser Verbindungen,

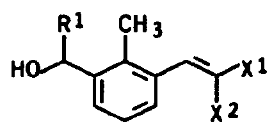

(V)

wobei A, $R^1$, $X^1$ und $X^2$ jeweils die in den Ansprüchen 1 bis 3 angegebene Bedeutung haben, bei Temperaturen von -20°C bis +150°C gegebenenfalls in Gegenwart eines Lösungsmittels, eines Katalysators, eines Säurebindemittels, eines Alkali- oder Erdalkalicyanids umsetzt.

5. Schädlingsbekämpfungsmittel, enthaltend feste oder flüssige Trägerstoffe und mindestens eine Verbindung gemäß den Ansprüchen 1 bis 3.

6. Schädlingsbekämpfungsmittel nach Anspruch 5, dadurch gekennzeichnet, daß es 0,01 bis 95 Gew.% eines Dihalogenvinyl-benzylesters der Formel I enthält.

7. Verfahren zur Bekämpfung von Schädlingen, dadurch gekennzeichnet, daß man Dihalogenvinyl-benzylester gemäß den Ansprüchen 1 bis 3 auf die Schädlinge bzw. deren Lebensraum einwirken läßt.

8. Benzylalkohole der allgemeinen Formel V

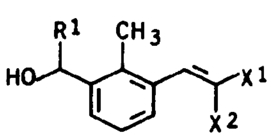

(V)

in der

$R^1$ Wasserstoff, Cyano, $C_1$-$C_4$-Alkyl, $C_2$-$C_4$-Alkenyl oder $C_2$-$C_4$-Alkinyl,
$X^1$ Chlor, Brom oder Fluor und
$X^2$ Chlor, Brom oder Fluor
bedeuten.

## Claims

1. A dihalovinylbenzyl ester of the general formula I

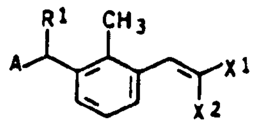

(I),

where $R^1$ is hydrogen, cyano, $C_1$-$C_2$-alkyl, $C_2$-$C_4$-alkenyl or $C_2$-$C_4$-alkynyl, $X^1$ and $X^2$ are each chlorine, bromine or fluorine, and A is the carboxylate radical of an acid component typical for pyrethroids.

2. A compound as claimed in claim 1, wherein $R^1$ is hydrogen.

3. A compound as claimed in claims 1 and 2, wherein A is a carboxylate radical II, III or IV

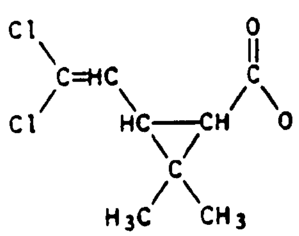

(II)

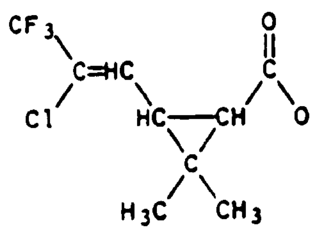

(III)

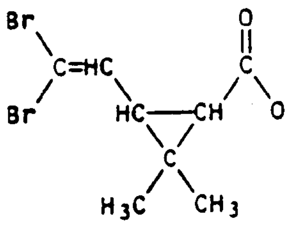

(IV).

4. A process for the preparation of compounds as claimed in claims 1 to 3, wherein a pyrethroid-typical acid of the general formula A-H, or an acid derivative thereof, is reacted with a compound of the general formula V or a derivative thereof

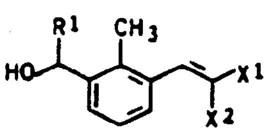

(V),

where A, $R^1$, $X^1$ and $X^2$ have the meanings given in claims 1 to 3, at from -20°C to +150°C in the presence or absence of a solvent, a catalyst, an acid acceptor and an alkali metal or alkaline earth metal cyanide.

5. A pesticide containing solid or liquid carriers and at least one compound as in claims 1 to 3.

6. A pesticide as claimed in claim 5 containing the dihalovinylbenzyl ester of the formula I in an amount of from 0.01 to 95% by weight.

7. A method for controlling pests, wherein a dihalovinylbenzyl ester as claimed in claims 1 to 3 is allowed to act on the pests or their habitat.

8. A benzyl alcohol of the general formula V

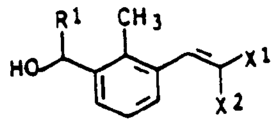

(V),

where $R^1$ is hydrogen, cyano, $C_1$-$C_4$-alkyl, $C_2$-$C_4$-alkenyl or $C_2$-$C_4$-alkynyl, $X^1$ is chlorine, bromine or fluorine and $X^2$ is chlorine, bromine or fluorine.

## Revendications

1. Esters dihalogénovinyl-benzyliques de formule générale I

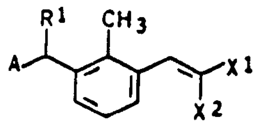

(I)

dans laquelle
$R^1$ représente l'hydrogène, un groupe cyano, alkyle en C1-C4, alcényle en C2-C4 ou alcynyle en C2-C4,
$X^1$ représente le chlore, le brome ou le fluor,
$X^2$ représente le chlore, le brome ou le fluor, et
A représente le radical carboxylate d'un composant acide typique des pyréthroïdes.

2. Composés selon la revendication 1, caractérisés en ce que $R^1$ représente l'hydrogène.

3. Composés selon les revendications 1 et 2, caractérisés en ce que A représente un radical carboxylate II, III ou IV :

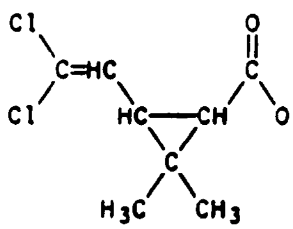

(II)

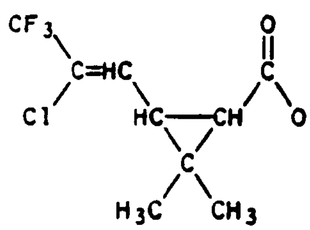

(III)

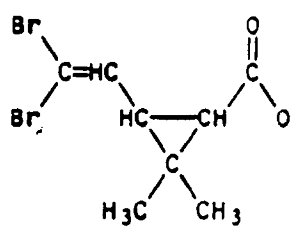

(IV)

4. Procédé de préparation des composés selon les revendications 1 à 3, caractérisé en ce que l'on fait réagir des acides typiques des pyréthroïdes, de formule générale A-H ou leurs dérivés avec des composés de formule générale V ou leurs dérivés

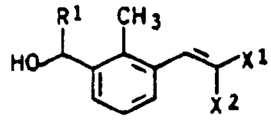

(V)

dans lesquels A, $R^1$, $X^1$ et $X^2$ ont les significations indiquées dans les revendications 1 à 3, à des températures de -20 à +150 degrés C, éventuellement en présence d'un solvant, d'un catalyseur, d'un accepteur d'acide, d'un cyanure alcalin ou alcalino-terreux.

5. Produit parasiticide, contenant des véhicules solides ou liquides et au moins un composé selon les revendications 1 à 3.

6. Produit parasiticides selon la revendication 5, caractérisé en ce qu'il contient de 0,01 à 95 % en poids d'un ester dihalogénovinyl-benzylique de formule I.

7. Procédé pour combattre les parasites, caractérisé en ce que l'on fait agir des esters dihalogénovinyl-benzyliques selon les revendications 1 à 3 sur les parasites ou leur habitat.

8. Alcools benzyliques de formule générale V

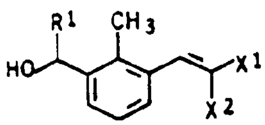

(V)

dans laquelle
$R^1$ représente l'hydrogène, un groupe cyano, alkyle en C1-C4, alcényle en C2-C4 ou alcynyle en C2-C4,
$X^1$ représente le chlore, le brome ou le fluor, et
$X^2$ représente le chlore, le brome ou le fluor.